# FASCICULE DE BREVET EUROPEEN

(11) **EP 0 648 508 B1**
(45) Date de publication et mention de la délivrance du brevet: **23.09.1998**
(21) Numéro de dépôt: 94420273.8
(22) Date de dépôt: 14.10.1994
(51) Int. Cl.: A61M 1/16, A61J 3/00

(54) **Dispositif d'entretien pour machine de dialyse**
Wartungseinrichtung für eine Dialysemaschine
Servicing apparatus for dialysis machine

(30) Priorité: 15.10.1993 FR 9312535
(43) Date de publication de la demande: 19.04.1995
(73) Titulaire: HOSPAL INDUSTRIE, F-69883 Meyzieu Cédex (FR)
(72) Inventeur: Chevallet, Jacques, F-69360 Serezin du Rhone (FR)
(74) Mandataire: Lejeune, Daniel

(56) Documents cités:
- EP-A- 0 491 981
- EP-A- 0 556 075
- EP-A- 0 556 098
- WO-A-92/11046
- DE-A- 3 447 989
- LU-A- 52 821
- US-A- 4 150 673

## Description

La présente invention concerne un dispositif d'entretien du circuit hydraulique d'une machine de traitement extracorporel de sang.

Le problème de l'entretien et notamment de la désinfection et du détartrage de circuit hydraulique se pose de façon particulièrement aiguë pour les circuits de liquide de dialyse des machines d'hémodialyse.

En effet, I'épuration du sang par dialyse implique des échanges entre le sang et un liquide épurateur ou liquide de dialyse. Bien que le contact sang / liquide de dialyse n'ait lieu qu'à travers une membrane, il est néanmoins essentiel que le liquide de dialyse soit dépourvu d'éléments susceptibles de contaminer le sang. Il est donc impératif que le circuit hydraulique à l'intérieur duquel circule ce liquide de dialyse soit parfaitement entretenu.

Or, les opérations d'entretien à réaliser sont de différentes natures. En effet, il est à la fois nécessaire de nettoyer le circuit, de le désinfecter et de le détartrer. L'opération de nettoyage consiste à supprimer les dépôts éventuels, tels que les dépôts protéiques et lipidiques, ces dépôts constituant des points d'ancrage pour les microorganismes. L'opération de désinfection consiste à détruire les microorganismes vivants. L'opération de détartrage a pour but l'élimination des dépôts calcaires provoqués notamment par des précipités de carbonates de calcium ou de magnésium, dus au calcium et au magnésium présents dans le liquide de dialyse. La réalisation de toutes ces opérations nécessite l'utilisation de différents produits. En effet, il n'existe pas de produit, dont l'utilisation permettrait de réaliser de façon parfaite à la fois le nettoyage, le détartrage et la désinfection. Aussi, les fabricants de machines d'hémodialyse recommandent-ils en général de varier les produits et procédures utilisés. Comme l'efficacité des produits utilisés est accrue lorsque le temps de contact à l'intérieur du circuit est prolongé, et qu'il est nécessaire après chaque cycle de rincer le circuit, ces opérations d'entretien représentent une part importante du temps de service du personnel hospitalier qui doit veiller à leur mise en route, leur bon déroulement et leur séquençage : ainsi, une opération de désinfection d'un circuit par de la vapeur d'eau risquant de provoquer la coagulation des dépôts organiques, il est nécessaire de la faire précéder d'un cycle de nettoyage, par exemple au moyen d'une solution aqueuse d'hypochlorite de sodium.

Une machine de dialyse équipée d'un système de désinfection pour le circuit de liquide de dialyse est connue par le document DE 34 47 989. Cette machine de dialyse comprend:
- un circuit de liquide de dialyse,
- des moyens de circulation de liquide dans le circuit de liquide de dialyse,
- un dispositif de désinfection du circuit de liquide de dialyse comprenant :
   - un réservoir pour la préparation d'un liquide de désinfection à partir d'eau et d'un désinfectant concentré liquide, connectable au circuit de liquide de dialyse,
   - des moyens pour introduire dans le réservoir le désinfectant concentré liquide, et
- des moyens de commande pour commander la circulation et la stagnation du liquide de désinfection dans le circuit de liquide de dialyse.

Le fait d'utiliser des solutions concentrées, ainsi que cela est généralement le cas pour ce genre d'opération, présente de nombreux inconvénients. D'une part, le risque d'erreur est important étant donné les dilutions à réaliser et la variété des solutions utilisées ; or, à moins d'utiliser une sonde spécifique à chaque produit à reconnaître, il est très difficile d'équiper une machine de dialyse d'un système de sécurité satisfaisant permettant de vérifier que le concentré utilisé est bien celui correspondant à l'action souhaitée. D'autre part, l'utilisation de solutions concentrées nécessite la manipulation et le stockage de réservoirs pouvant être encombrants, et entraîne également des difficultés de gestion des stocks en fonction des dates de péremption. Dans cet ordre d'idée, le document WO 92/11046 propose de préparer un liquide de dialyse extemporanément non plus à partir de solutions concentrées liquides mais de pastilles d'acide, de sels, et de base.

La présente invention a pour but de pallier les inconvénients des machines de dialyse classiques mentionnés plus haut et de supprimer une part importante des contraintes liées à un entretien correct des circuits hydrauliques des dispositifs de traitement extracorporel de sang.

L'invention a également pour but de proposer un système de préparation et de mise en oeuvre automatiques de différents liquides d'entretien.

Pour atteindre ce but, l'invention propose une machine d'hémodialyse/hémofiltration comprenant :
- un dispositif d'entretien du circuit de liquide de dialyse comprenant :
   - un réservoir pour la préparation d'au moins un liquide d'entretien à partir d'eau et d'une substance concentrée, connectable au circuit de liquide de dialyse,
   - des moyens pour introduire dans le réservoir la substance concentrée,
- des moyens de commande pour commander la circulation et la stagnation d'un liquide d'entretien dans le circuit de liquide de dialyse,
   caractérisée en ce que les moyens pour introduire dans le réservoir une substance concentrée comportent :
- au moins un magasin pour contenir au moins un comprimé de substance concentrée, et
- des moyens de communication obturables entre le magasin et le réservoir pour permettre l'introduction, sur commande, d'un comprimé de substance concentrée dans le réservoir.

Selon une caractéristique de l'invention, le magasin est prévu pour contenir une pluralité de comprimés et les moyens de commande sont prévus pour commander l'introduction successive de au moins deux comprimés de nature chimique différente dans le réservoir ainsi que la préparation successive des liquides d'entretien correspondants, des comprimés de nature chimique différente étant alors disposés dans le magasin dans un ordre correspondant à une succession de cycles d'entretien prédéterminée.

Selon une autre caractéristique de l'invention, le dispositif d'entretien comporte au moins deux magasins et chaque magasin comprend des moyens d'accès sélectifs pour n'autoriser l'introduction dans ce magasin que de comprimés de même nature chimique. Dans un mode de réalisation, les moyens d'accès comprennent des moyens de détrompage géométrique complémentaires d'au moins une caractéristique géométrique d'un comprimé de nature chimique spécifique.

D'autres caractéristiques et avantages de la présente invention apparaîtront à la lecture de la description qui va suivre. On se reportera aux figures annexées sur lesquelles :
La figure 1 représente un premier mode de réalisation d'un dispositif d'entretien selon la présente invention.
La figure 2 représente un second mode de réalisation d'un dispositif d'entretien selon l'invention.

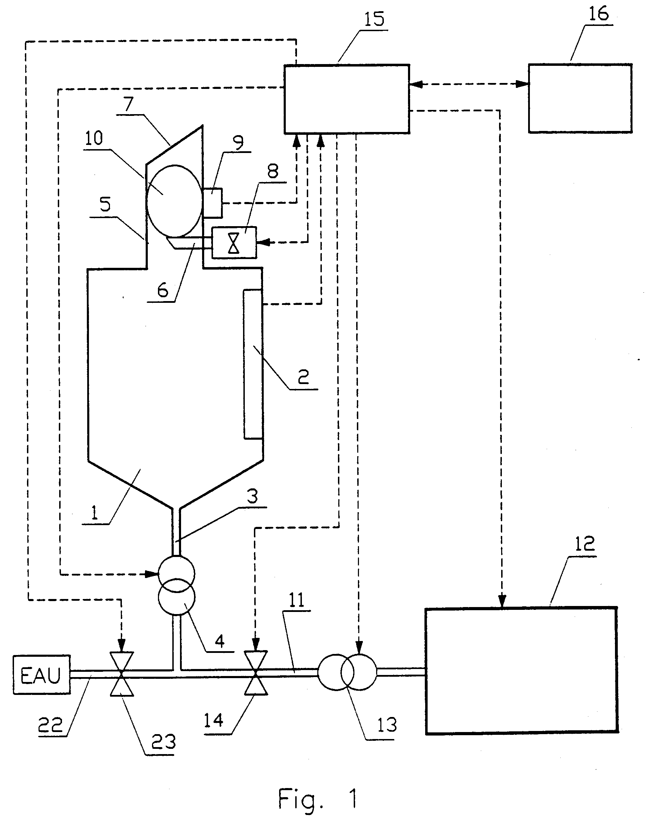

Ainsi que cela est représenté sur la figure 1, le dispositif d'entretien selon l'invention comprend une unité de mélange constituée par un réservoir 1 pourvu d'un détecteur de niveau 2. Le réservoir 1 est relié, par sa partie inférieure présentant une forme d'entonnoir, à une canalisation 3 pourvue d'une pompe 4. Cette canalisation 3 est reliée par une canalisation 22 munie d'une vanne 23 à une source d'eau, et par une canalisation 11, à un circuit de liquide de dialyse représenté ici globalement par le bloc 12. Il est intéressant de placer le dispositif de préparation selon l'invention le plus en amont possible par rapport à la source d'eau, de manière à réaliser les opérations d'entretien sur la totalité du circuit hydraulique. La canalisation 11 est munie d'une pompe de circulation 13 et d'une vanne d'arrêt 14 située en amont de la pompe 13. Bien que pour des raisons de simplification, cette canalisation 11 soit représentée ici comme étant une canalisation de liaison entre le réservoir 1 et le circuit de liquide de dialyse, il est possible qu'elle fasse en réalité partie du circuit de liquide de dialyse, la pompe 13 étant alors une des pompes du circuit. Le dispositif d'entretien est également muni d'un distributeur 5 de comprimés 10 à dissoudre dans de l'eau. Le distributeur 5 comprend un magasin pour le stockage d'au moins un comprimé, des moyens d'accès sélectifs au magasin tels qu'une fente 7 d'introduction de comprimé et des moyens de communication obturables entre le magasin et le réservoir, comprenant un doigt mobile 6 mû par un électroaimant 8. Le distributeur 5 comporte également un détecteur-décodeur 9 apte à détecter la présence du comprimé 10, et éventuellement à reconnaître sa nature, grâce à la lecture d'un code-barres présent sur le comprimé par exemple.

Une unité de commande 15, constituée, par exemple, par un microprocesseur, reçoit des informations provenant du détecteur de niveau 2 et du détecteur-décodeur 9 ; cette unité 15 est également reliée aux pompes 4 et 13, aux vannes 14 et 23, à l'électroaimant 8 ainsi qu'à un certain nombre d'éléments du circuit de liquide de dialyse 12 dont elle commande le fonctionnement. De plus, elle possède une mémoire dans laquelle sont enregistrées les données relatives aux différents cycles de préparation et de circulation du liquide d'entretien. Elle est, en outre, reliée à une interface utilisateur 16.

Le fonctionnement du dispositif ainsi décrit est le suivant. Lorsque l'utilisateur prévoit d'effectuer un cycle d'entretien particulier, il indique à l'unité de contrôle 15 via l'interface 16, le cycle choisi : détartrage, désinfection, nettoyage, etc... et il introduit dans le distributeur 5 un comprimé.

Le détecteur-décodeur 9 détecte alors la présence d'un comprimé et identifie sa nature, par exemple par lecture du code-barres si c'est le système d'identification choisi, ou par reconnaissance de la couleur du comprimé dans le cas où les comprimés de nature différente sont identifiés par des couleurs différentes. Les informations relatives à la présence et à la nature du comprimé introduit sont transmises à l'unité 15 qui les compare avec le cycle d'entretien sélectionné. Dans le cas où la nature du comprimé ne correspond pas au cycle d'entretien sélectionné, un signal est transmis à l'utilisateur qui peut alors modifier le cycle choisi pour le faire correspondre avec la nature du comprimé introduit. Alternativement, il est possible de munir le distributeur 5 d'une ouverture (non représentée) permettant de rejeter un comprimé ne correspondant pas au cycle sélectionné. Dans le cas où le comprimé introduit est de nature à permettre le cycle d'entretien choisi, l'unité 15 commande l'électroaimant 8 qui agit sur l'organe 6 pour le rétracter et libérer ainsi le comprimé 10 ; ensuite l'unité 15 commande la fermeture de la vanne 14 si cela est nécessaire, l'ouverture de la vanne 23, ainsi que la mise en oeuvre de la pompe 4 afin d'alimenter le réservoir 1 en eau provenant de la canalisation 22. Le niveau d'eau à l'intérieur du réservoir 1 est transmis, grâce au détecteur 2, à l'unité 15 qui commande l'arrêt de la pompe 4 lorsque le niveau atteint correspond à la quantité nécessaire pour dissoudre le comprimé introduit et effectuer le cycle d'entretien choisi. Durant un temps d'attente déterminé correspondant à une bonne dissolution du comprimé, et pouvant éventuellement être favorisé par la mise en oeuvre de moyens d'agitation (non représentés) à l'intérieur du réservoir 1, les vannes 23 et 14 peuvent être ouvertes et la pompe 13 mise en oeuvre afin d'assurer le rinçage du circuit de liquide de dialyse. Lorsque la dissolution du comprimé introduit est totale, l'unité 15 commande la mise en oeuvre de la pompe 4 pour alimenter la canalisation de liaison 11. Les débits des pompes 4 et 13 sont réglés pour effectuer une dilution adéquate du liquide préparé dans le réservoir 1 ; alternativement, si la quantité et la concentration du liquide à l'intérieur du réservoir 1 conviennent au remplissage du circuit de liquide de dialyse, la vanne 23 est fermée et les pompes 4 et 13 fonctionnent au même débit. Les divers éléments du circuit de liquide de dialyse (pompes, vannes...) sont en outre commandés pour permettre une action optimale du produit préparé. Ainsi, l'unité 15 commande le fonctionnement des différentes pompes pour assurer le remplissage du circuit de liquide de dialyse avec le liquide provenant du réservoir 1 ; puis le fonctionnement des pompes est arrêté, le liquide restant présent dans le circuit pendant la durée nécessaire à son action, durée qui varie selon la nature du liquide utilisé et l'effet recherché. Ainsi, lorsqu'il s'agit d'un liquide à action détartrante obtenu par dissolution dans de l'eau d'un comprimé d'acide citrique, le temps de contact dans le circuit à détartrer est par exemple de 10 à 15 min. Par contre, lorsqu'il s'agit d'un liquide à action désinfectante obtenu par dissolution dans de l'eau d'un comprimé de sel de sodium de dichloro-S-triazinetrione, le temps de contact dans le circuit peut être de 15 à 20 min. Lorsque le temps de contact correspondant au cycle d'entretien sélectionné est écoulé, l'unité 15 agit sur les éléments du circuit de liquide de dialyse 12 pour vidanger le circuit, puis le rincer soit grâce à de l'eau mise en circulation au moyen de la pompe 13 et l'ouverture des vannes 14 et 23, soit au moyen d'un liquide stérile (non représenté). Le circuit de liquide de dialyse est alors prêt à subir un autre cycle d'entretien avec un liquide de nature différente, ou à recevoir du liquide de dialyse.

Alternativement à ce qui vient d'être décrit, il est possible que l'utilisateur n'indique pas à l'unité de contrôle 15 le cycle d'entretien choisi, mais se contente d'introduire dans le distributeur 5, le comprimé approprié. Dans ce cas, le détecteur-décodeur 9 identifie le comprimé introduit et transmet l'information relative à sa nature à l'unité de contrôle 15 qui commande alors l'exécution du programme permettant la réalisation de l'opération d'entretien correspondant au comprimé introduit.

Il est possible également de prévoir l'obligation pour l'utilisateur d'effectuer les cycles d'entretien selon une séquence préétablie, en introduisant dans l'unité de contrôle 15 un programme vérifiant par exemple qu'après chaque séance d'hémodialyse, un cycle de désinfection avec un comprimé de sel de sodium de dichloro-S-triazinetrione est réalisé, qu'un cycle de désinfection à la chaleur est réalisé en alternance avec le sel de sodium de dichloro-S-triazinetrione au moins une fois par jour et, qu'un cycle de détartrage avec un comprimé d'acide citrique est effectué au moins une fois par semaine. Dans un tel cas, il est possible de prévoir entre le distributeur 5 et le réservoir 1 un conduit permettant l'évacuation du comprimé par l'utilisateur s'il ne convient pas pour effectuer le cycle d'entretien programmé.

Dans le dispositif représenté sur la figure 1, le magasin du distributeur 5 ne contient qu'un comprimé, de sorte que chaque cycle d'entretien (détartrage, nettoyage/désinfection), requiert de l'utilisateur qu'il introduise un comprimé de composition chimique correspondante. Dans une variante de réalisation, le magasin peut contenir plusieurs comprimés. Pourvu que des comprimés de nature différente y soient introduits selon une séquence déterminée, le dispositif peut alors être programmé pour effectuer automatiquement plusieurs cycles d'entretien différents successifs. L'organe de retenue 6 est alors adapté pour ne libérer qu'un comprimé à la fois. Ainsi, lorsqu'un cycle d'entretien a été effectué tel que cela a été décrit précédemment, le détecteur-décodeur 9 identifie le second comprimé introduit et transmet à l'unité 15, les informations relatives à la nature de ce second comprimé : un nouveau cycle d'entretien correspondant à la nature de ce second comprimé est alors effectué. Il est possible de cette manière d'exécuter par exemple un cycle de détartrage suivi d'un cycle de désinfection sans intervention autre de la part de l'utilisateur que l'approvisionnement préalable du distributeur 5 en comprimés appropriés.

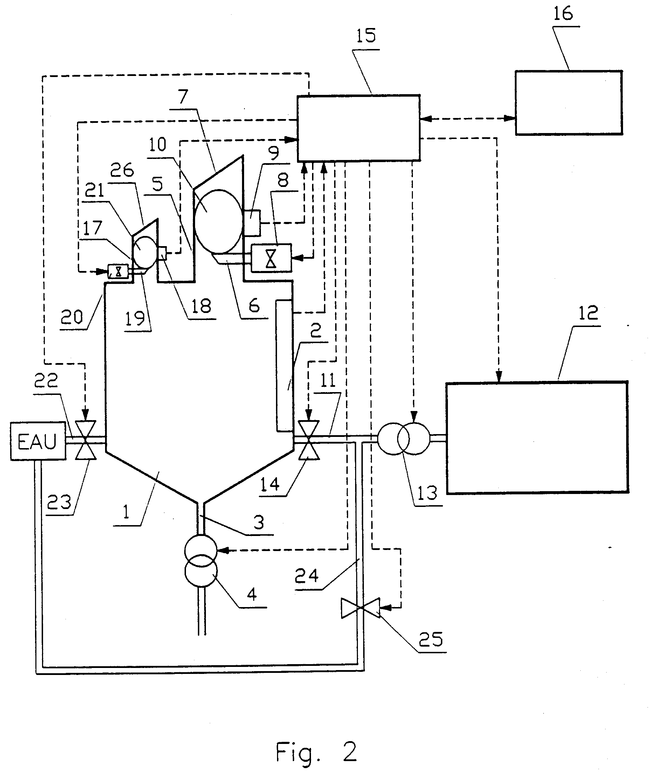

Selon le mode de réalisation représenté sur la figure 2, sur laquelle les éléments identiques à ceux de la figure 1 sont indiqués par les mêmes numéros de référence, le réservoir 1 est muni, non pas d'un seul distributeur 5, mais de 2 distributeurs adaptés pour ne recevoir qu'un type déterminé de comprimé. Dans ce mode de réalisation, à la nature chimique des comprimés est associée une forme spécifique (épaisseur, diamètre des comprimés). Ainsi, au distributeur 5 est adjoint un second distributeur 17, muni de la même façon que le distributeur 5 d'un détecteur-décodeur 18 ainsi que d'un organe de retenue 19 actionné par un électroaimant 20. Le détecteur 18 et l'électroaimant 20 sont reliés à l'unité de contrôle 15 de la même façon que le détecteur 9 et l'électroaimant 8. Le distributeur 17 est pourvu d'une ouverture 26 constituée par une fente d'introduction dont la forme correspond à la forme du comprimé 21 que l'on souhaite y introduire. Un tel détrompage permet d'éviter des erreurs de manipulation de comprimé de la part de l'utilisateur. Selon ce mode de réalisation, la canalisation 3 située à la base du réservoir 1 n'est plus reliée à la canalisation 22 d'alimentation en eau ni à la canalisation 11 de liaison avec le circuit de liquide de dialyse, mais à des moyens d'évacuation (non représentés), ce qui permet l'élimination des éléments insolubles. Les canalisations 22 et 11 sont donc reliées au réservoir 1 indépendamment l'une de l'autre. Une canalisation 24, munie d'une vanne 25 commandée par l'unité 15 relie la canalisation 11, en un point situé en amont de la pompe 13 et en aval de la vanne 14, à une source d'eau.
Le fonctionnement de ce dispositif est le suivant :

L'utilisateur voulant faire exécuter deux cycles d'entretien de nature différente, par exemple un cycle de détartrage suivi d'un cycle de désinfection, introduit dans le distributeur 5 un comprimé dont la nature correspond à un des cycles (par exemple le détartrage) et introduit dans le distributeur 17 un autre comprimé dont la nature correspond à l'autre cycle (par exemple la désinfection) ; ces comprimés sont maintenus en attente dans les distributeurs grâce aux organes de retenue 6 et 19. Il indique en outre à l'unité de contrôle 15, via l'interface 16, la séquence sélectionnée pour les opérations d'entretien à effectuer. A partir de cet instant, l'ensemble des opérations peut se dérouler automatiquement, sans intervention de l'utilisateur. Ainsi, I'unité de contrôle 15 vérifie grâce aux informations transmises par les détecteurs-décodeurs 9 et 18 que les distributeurs 5 et 17 ont bien été alimentés. En fait, étant donné la présence d'un détrompage ne permettant d'introduire dans un distributeur que le comprimé de forme appropriée, il n'est pas indispensable, dans ce mode de réalisation, que les détecteurs 9 et 18 soient également capables d'exercer la fonction d'identification de la nature du comprimé introduit. Lorsque la vérification de la présence des comprimés a été effectuée, I'unité 15 ferme la vanne 14 et commande l'électroaimant 8 pour agir sur l'organe de retenue 6 et libérer le comprimé-détartrant 10. Celui-ci tombe dans le réservoir 1. L'unité 15 commande alors le cycle de préparation de la solution détartrante et sa circulation dans le circuit de liquide de dialyse 12, ainsi que cela a été décrit en relation avec le mode de réalisation de la figure 1. Cependant, de façon avantageuse, pendant le temps de dissolution du comprimé-détartrant, I'unité 15 commande le rinçage du circuit 12 grâce à l'ouverture de la vanne 25 et la mise en oeuvre de la pompe 13. De même, pendant le temps de contact de la solution détartrante à l'intérieur du circuit, I'unité 15 commande la mise en oeuvre de la pompe 4 afin d'éliminer les éléments non solubles présents dans le fond du réservoir 1. Lorsque le cycle de détartrage a été effectué, I'unité 15 commande la fermeture de la vanne 14 ainsi que la mise en oeuvre de la pompe 13 afin d'effectuer à nouveau un rinçage du circuit. Pendant ce temps, I'électroaimant 20 est commandé pour agir sur l'organe de retenue 19 et libérer le comprimé désinfectant 21 qui tombe alors dans le réservoir 1. L'unité 15 commande alors l'exécution du cycle de préparation de la solution désinfectante et sa circulation dans le circuit de liquide de dialyse 12, de façon similaire à ce qui a été décrit en relation avec le mode de réalisation de la figure 1 ; cependant, de même que pour la solution détartrante, après que l'essentiel du contenu du réservoir 1 a été mis en circulation dans le circuit 12, I'unité 15 commande la fermeture de la vanne 14 et la mise en oeuvre de la pompe 4 afin d'éliminer les éléments insolubles présents dans le fond du réservoir. L'action de la solution désinfectante étant généralement d'autant plus efficace que le temps de contact est long, il est possible de la maintenir dans le circuit jusqu'à la séance de traitement suivante, à condition que le désinfectant utilisé n'ait aucune activité corrosive vis-à-vis des éléments avec lesquels il est en contact.

Selon le mode de réalisation décrit, I'unité de mélange constituée par le réservoir 1 est alimentée par deux distributeurs ; il est bien évident qu'il est possible d'en prévoir plus, chacun d'eux étant muni d'un détrompage ne permettant que l'introduction du comprimé approprié. Dans ce cas, il est possible de réaliser un cycle d'entretien complet impliquant la mise en oeuvre successive de chacun des cycles d'entretien particuliers correspondant aux comprimés présents dans chaque distributeur, suivi d'un rinçage du circuit ; alternativement, l'utilisateur peut choisir de n'effectuer qu'un seul cycle d'entretien, par exemple un cycle de désinfection : dans ce cas, il alimente le distributeur approprié et indique à l'unité de contrôle 15, via l'interface 16, le cycle sélectionné. L'unité de contrôle 15 vérifie alors que le distributeur correspondant au cycle sélectionné est alimenté : si ce n'est pas le cas, un signal d'avertissement est émis ; si tout est en ordre, I'unité de contrôle 15 commande l'exécution du programme correspondant au cycle choisi. Ainsi que cela a été décrit en relation avec le mode de réalisation précédent, il est également possible ici d'obliger l'utilisateur à varier la nature des cycles d'entretien selon une séquence préétablie.

Alternativement à ce qui vient d'être décrit, il est possible que le liquide préparé dans le réservoir 1 soit dilué avant sa mise en circulation dans le circuit 12. Dans ce cas, il est possible de disposer sur la canalisation 11, en amont de l'intersection avec la canalisation 24 une pompe supplémentaire dont le débit sera réglé pour que le rapport de débit entre cette pompe et la pompe 13 corresponde au taux de dilution souhaité. Lors du transfert du liquide du réservoir 1 au circuit 12, la vanne 25 sera ouverte pour permettre la dilution de la solution préparée.

La présente invention est susceptible de nombreuses variantes de réalisation. Ainsi, bien que selon la description qui précède, les distributeurs soient adaptés pour que les comprimés introduits soient maintenus dans un plan vertical, il est également possible de les maintenir dans un plan horizontal.

On peut également munir les distributeurs d'un ou plusieurs clapets permettant de les isoler par rapport au contenu du réservoir.

En outre, il est possible que le produit destiné à être dissous dans le réservoir 1 ne se présente pas sous forme de comprimé mais sous forme de poudre dont la nature peut être identifiée par la couleur ou par la densité.

La présente invention a été décrite en relation avec un circuit de liquide de dialyse d'un rein artificiel ; elle est cependant susceptible d'application pour l'entretien de tout circuit hydraulique d'une machine de traitement extracorporel de sang.

Parmi les avantages apportés par la présente invention, le détrompage géométrique ou l'association des détecteurs-décodeurs avec un dispositif d'évacuation des comprimés non appropriés, constituent un dispositif d'alimentation sélective du produit à dissoudre en relation avec le cycle d'entretien sélectionné qui permet d'assurer à l'utilisateur un haut niveau de fiabilité.

## Revendications

1. Machine d'hémodialyse/hémofiltration comprenant :
- un circuit de liquide de dialyse (12),
- des moyens de circulation de liquide (13) dans le circuit de liquide de dialyse (12),
- un dispositif d'entretien du circuit de liquide de dialyse comprenant :
• un réservoir (1) pour la préparation d'au moins un liquide d'entretien à partir d'eau et d'une substance concentrée, connectable au circuit de liquide de dialyse (12),
• des moyens (5, 6, 7, 8; 17, 19, 20, 26) pour introduire dans le réservoir (1) la substance concentrée, et
- des moyens de commande (15) pour commander la circulation et la stagnation d'un liquide d'entretien dans le circuit de liquide de dialyse (12) ;
caractérisée en ce que les moyens pour introduire dans le réservoir (1) une substance concentrée comportent :
- au moins un magasin (5, 6; 17, 26) pour contenir au moins un comprimé (10, 21)) de substance concentrée, et
- des moyens de communication obturables (6, 8; 19, 20) entre le magasin (5, 6; 17, 26) et le réservoir (1) pour permettre l'introduction, sur commande, d'un comprimé de substance concentrée dans le réservoir (1).

2. Machine d'hémodialyse/hémofiltration selon la revendication 1, caractérisée en ce que le magasin (5, 6; 17, 26) est prévu pour contenir une pluralité de comprimés (10, 21).

3. Machine d'hémodialyse/hémofiltration selon la revendication 2, caractérisée en ce que les moyens de commande (15) sont prévus pour commander l'introduction successive de au moins deux comprimés (10, 21) de nature chimique différente dans le réservoir (1) et la préparation successive des liquides d'entretien correspondants, des comprimés (10, 21) de nature chimique différente étant alors disposés dans le magasin (5, 6; 17, 26) dans un ordre correspondant à une succession de cycles d'entretien prédéterminée.

4. Machine d'hémodialyse/hémofiltration selon une des revendications 1 et 2, caractérisée en ce que le dispositif d'entretien comporte au moins deux magasins (5, 6; 17, 26) pour comprimés (10, 21) de nature chimique différente.

5. Machine d'hémodialyse/hémofiltration selon la revendication 4, caractérisée en ce que les moyens de commande (15) sont prévus pour commander l'introduction successive dans le réservoir (1) de au moins deux comprimés (10, 21) de nature chimique différente et la préparation successive des liquides d'entretien correspondants.

6. Machine d'hémodialyse/hémofiltration selon une des revendications 4 et 5, caractérisée en ce que chaque magasin (5, 6; 17, 26) comprend des moyens d'accès (7, 26) sélectifs pour n'autoriser l'introduction dans ce magasin que de comprimés (10, 21) de même nature chimique.

7. Machine d'hémodialyse/hémofiltration selon la revendication 6, caractérisée en ce que les moyens d'accès sélectifs comprennent des moyens de détrompage géométrique (7, 26) complémentaires d'au moins une caractéristique géométrique d'un comprimé (10, 21) de nature chimique spécifique.

8. Machine d'hémodialyse/hémofiltration selon une des revendications 1 à 7, caractérisée en ce que le dispositif d'entretien comprend des moyens (9; 18) de détection de la nature chimique des comprimés (10, 21) stockés dans le magasin (5, 6; 17, 26).

9. Machine d'hémodialyse/hémofiltration selon une des revendications 1 à 8, caractérisée en ce que le dispositif d'entretien comprend des moyens (3, 4) d'évacuation d'éléments insolubles.

10. Machine d'hémodialyse/hémofiltration selon une des revendications 1 à 9, caractérisée en ce qu'elle comprend des moyens pour vérifier la correspondance entre un cycle d'entretien déterminé et la nature chimique d'un comprimé (10, 21) introduit dans le réservoir (1)

## Claims

1. Haemodialysis/haemofiltration machine comprising:
- a dialysis liquid circuit (12),
- means (13) for circulation of liquid in the dialysis liquid circuit (12),
- a device for servicing the dialysis liquid circuit, comprising:
• a reservoir (1) for preparing at least one servicing liquid from water and a concentrated substance, connectable to the dialysis liquid circuit (12),
• means (5, 6, 7, 8; 17, 19, 20, 26) for introducing the concentrated substance into the reservoir (1), and
- control means (15) for controlling the circulation and the stagnation of a servicing liquid in the dialysis liquid circuit (12);
characterized in that the means for introducing a concentrated substance into the reservoir (1) include:
- at least one magazine (5, 6; 17, 26) for holding at least one tablet (10, 21) of concentrated substance, and
- closable means of communication (6, 8; 19, 20) between the magazine (5, 6; 17, 26) and the reservoir (1) to permit the introduction, on command, of a tablet of concentrated substance into the reservoir (1).

2. Haemodialysis/haemofiltration machine according to Claim 1, characterized in that the magazine (5, 6; 17, 26) is intended to hold a plurality of tablets (10, 21).

3. Haemodialysis/haemofiltration machine according to Claim 2, characterized in that the control means (15) are intended to control the successive introduction of at least two tablets (10, 21) of different chemical nature into the reservoir (1) and the successive preparation of the corresponding servicing liquids, tablets (10, 21) of different chemical nature then being arranged in the magazine (5, 6; 17, 26) in an order corresponding to a predetermined succession of servicing cycles.

4. Haemodialysis/haemofiltration machine according to one of Claims 1 and 2, characterized in that the servicing device includes at least two magazines (5, 6; 17, 26) for tablets (10, 21) of different chemical nature.

5. Haemodialysis/haemofiltration machine according to Claim 4, characterized in that the control means (15) are intended to control the successive introduction into the reservoir (1) of at least two tablets (10, 21) of different chemical nature and the successive preparation of the corresponding servicing liquids.

6. Haemodialysis/haemofiltration machine according to one of Claims 4 and 5, characterized in that each magazine (5, 6; 17, 26) comprises selective access means (7, 26) in order only to authorize the introduction, into this magazine, of tablets (10, 21) of the same chemical nature.

7. Haemodialysis/haemofiltration machine according to Claim 6, characterized in that the selective access means comprise additional geometric keying means (7, 26) of at least one geometric characteristic of a tablet (10, 21) of specific chemical nature.

8. Haemodialysis/haemofiltration machine according to one of Claims 1 to 7, characterized in that the servicing device comprises means (9; 18) for detecting the chemical nature of the tablets (10, 21) stocked in the magazine (5, 6; 17, 26).

9. Haemodialysis/haemofiltration machine according to one of Claims 1 to 8, characterized in that the servicing device comprises means (3, 4) for removal of insoluble elements.

10. Haemodialysis/haemofiltration machine according to one of Claims 1 to 9, characterized in that it comprises means for checking the correspondence between a determined servicing cycle and the chemical nature of a tablet (10, 21) introduced into the reservoir (1).

## Patentansprüche

1. Hämodialyse/Hämofiltrationsmaschine, die folgendes umfaßt:
- einen Dialyseflüssigkeitskreislauf (12),
- Flüssigkeitszirkulierungsmittel (13) im Dialyseflüssigkeitskreislauf (12),
- eine Wartungseinrichtung für den Dialyseflüssigkeitskreislauf mit
• einem an den Dialyseflüssigkeitskreislauf (12) anschließbaren Reservoir (1) für die Herstellung mindestens einer Wartungsflüssigkeit aus Wasser und einer konzentrierten Substanz und
• Mitteln (5, 6, 7, 8; 17, 19, 20, 26) zur Einführung der konzentrierten Substanz in das Reservoir (1), und
- Steuermittel (15) zum Steuern des Zirkulierens und des Ruhens einer Wartungsflüssigkeit im Dialyseflüssigkeitskreislauf (12),
dadurch gekennzeichnet, daß die Mittel zur Einführung einer konzentrierten Substanz in das Reservoir (1) folgendes umfassen:
- mindestens ein Magazin (5, 6; 17, 26), in dem mindestens eine Tablette (10, 21) aus konzentrierter Substanz enthalten ist, und
- absperrbare Verbindungsmittel (6, 8; 19, 20) zwischen dem Magazin (5, 6; 17, 26) und dem Reservoir (1), um die befehlsgemäße Einführung einer Tablette aus konzentrierter Substanz in das Reservoir (1) zu gestatten.

2. Hämodialyse/Hämofiltrationsmaschine nach Anspruch 1, dadurch gekennzeichnet, daß das Magazin (5, 6; 17, 26) dafür vorgesehen ist, mehrere Tabletten (10, 21) zu enthalten.

3. Hämodialyse/Hämofiltrationsmaschine nach Anspruch 2, dadurch gekennzeichnet, daß die Steuermittel (15) dafür vorgesehen sind, die aufeinanderfolgende Einführung von mindestens zwei Tabletten (10, 21) unterschiedlicher chemischer Beschaffenheit in das Reservoir (1) und die aufeinanderfolgende Herstellung der entsprechenden Wartungsflüssigkeiten zu steuern, wobei Tabletten (10, 21) unterschiedlicher chemischer Beschaffenheit in einer Abfolge in dem Magazin (5, 6; 17, 26) angeordnet sind, wie sie einer vorbestimmten Abfolge von Wartungszyklen entspricht.

4. Hämodialyse/Hämofiltrationsmaschine nach Anspruch 1 oder 2, dadurch gekennzeichnet, daß die Wartungseinrichtung mindestens zwei Magazine (5, 6; 17, 26) für Tabletten (10, 21) unterschiedlicher chemischer Beschaffenheit umfaßt.

5. Hämodialyse/Hämofiltrationsmaschine nach Anspruch 4, dadurch gekennzeichnet, daß die Steuermittel (15) dafür vorgesehen sind, die aufeinanderfolgende Einführung mindestens zweier Tabletten (10, 21) unterschiedlicher chemischer Beschaffenheit in das Reservoir (1) und die aufeinanderfolgende Herstellung der entsprechenden Wartungsflüssigkeiten zu steuern.

6. Hämodialyse/Hämofiltrationsmaschine nach Anspruch 4 oder 5, dadurch gekennzeichnet, daß jedes Magazin (5, 6; 17, 26) gezielte Zutrittsmittel (7, 26) umfaßt, so daß nur die Einführung von Tabletten (10, 21) der gleichen chemischen Beschaffenheit in dieses Magazin gestattet wird.

7. Hämodialyse/Hämofiltrationsmaschine nach Anspruch 6, dadurch gekennzeichnet, daß die gezielten Zutrittsmittel Mittel der geometrischen Unverwechselbarkeit (7, 26) umfassen, die mindestens einer geometrischen Eigenschaft einer Tablette (10, 21) einer spezifischen chemischen Beschaffenheit komplementär sind.

8. Hämodialyse/Hämofiltrationsmaschine nach einem der Ansprüche 1 bis 7, dadurch gekennzeichnet, daß die Wartungseinrichtung Mittel (9; 18) zur Erfassung der chemischen Beschaffenheit der in dem Magazin (5, 6; 17, 26) gelagerten Tabletten (10, 21) umfaßt.

9. Hämodialyse/Hämofiltrationsmaschine nach einem der Ansprüche 1 bis 8, dadurch gekennzeichnet, daß die Wartungseinrichtung Mittel (3, 4) zur Entfernung unlöslicher Elemente umfaßt.

10. Hämodialyse/Hämofiltrationsmaschine nach einem der Ansprüche 1 bis 9, dadurch gekennzeichnet, daß sie Mittel zur Prüfung der Entsprechung zwischen einem bestimmten Wartungszyklus und der chemischen Beschaffenheit einer in das Reservoir (1) eingeführten Tablette (10, 21) umfaßt.
